Europäisches Patentamt

European Patent Office

Office européen des brevets

(19)

(11) Numéro de publication: **0 134 729**
**B1**

(12) **FASCICULE DE BREVET EUROPEEN**

(45) Date de publication du fascicule du brevet: **07.10.87**

(51) Int. Cl.⁴: **C 07 K 3/08, A 61 K 37/14**

(21) Numéro de dépôt: **84401302.9**

(22) Date de dépôt: **21.06.84**

(54) **Procédé de préparation de globine à partir d'hémoglobine et globine obtenue par ce procédé.**

(30) Priorité: **07.07.83 FR 8311324**

(43) Date de publication de la demande:
**20.03.85 Bulletin 85/12**

(45) Mention de la délivrance du brevet:
**07.10.87 Bulletin 87/41**

(84) Etats contractants désignés:
**AT BE CH DE GB IT LI LU NL SE**

(56) Documents cités:
**FR-A-2 274 230**
**FR-A-2 351 604**
**US-A-2 466 710**

(73) Titulaire: **INSTITUT MERIEUX Société anonyme dite:**
**17, rue Bourgelat**
**F-69002 LYON (FR)**

(72) Inventeur: **Tayot, J.L.**
**11 rue des Greffières**
**F-69890 La Tour de Salvagny (FR)**
Inventeur: **Veron, Jean-Luc Bernard**
**3 Boulevard de Jomardière**
**F-38120 Saint-Egrève (FR)**

(74) Mandataire: **Bernasconi, Jean et al**
**Cabinet Michel Lemoine 13 Boulevard des Batignolles**
**F-75008 Paris (FR)**

Courier Press, Leamington Spa, England.

EP 0 134 729 B1

**Description**

La présente invention a trait à un nouveau procédé de préparation de globine à partir d'hémoglobine ainsi qu'à la globine obtenue par ce procédé.

Les travaux de Anson et Mirsky (*J. Gen. Physiol. 13,469—476*) publiés en 1930 ont montré qu'il est possible de séparer le pigment de l'hémoglobine (hème) par la technique dite à l'acétone acide. On a ainsi déjà transformé en globine différentes hémoglobines humaines ou animales. Deux variantes de ce procédé sont généralement utilisées à l'échelle du laboratoire, toutes deux basées sur la faible affinité de l'hème pour la globine à pH acide.

L'un des procédés est la méthode à l'acétone acide (voir par exemple *Rossi-Fanelli et coll. (1958) Biophys. Bioch. Acta 30, 608—615.* Ce procédé consiste à ajouter un volume d'hémoglobine à 30 g/l à 20 à 30 volumes d'acétone à —20°C contenant 3 ml d'HCl 2N par litre, à agiter à —20°C puis centrifuger, le précipité de globine étant redissous en eau et dialysé contre de l'eau distillée.

Cette solution de globine n'est stable que lorsque son pH reste acide de sorte qu'il à été proposé (*STRUMIA et coll. J. of Lab. and Clin. Medicine, 37,6 959—968 en 1951*) une hydrolyse douce de la globine à un pH alcalin permettant de rendre la molécule soluble à pH neutre.

Le deuxième procédé est le procédé à la butanone-acide selon *TEALE 1959 Bioch. Biophys. Acta 35,543.* La substitution de l'acétone par la butanone, peu soluble dans l'eau, permet une séparation rapide en deux phases de l'hème (phase cétonique) et de la globine (phase aqueuse).

Ces deux procédés ne se prêtent cependant pas à une application industrielle en raison des volumes considérables de solvants organiques qu'il faudrait employer et des risques d'accidents que cela entraîne. En outre la globine finale obtenue contient des quantités non négligeables de résidus chimiques dangereux pour son utilisation alimentaire, dans le cas de la globine animale, ou thérapeutique, dans le cas de la globine humaine.

En conséquence, on a déjà proposé un certain nombre d'autres procédés dans le but d'obtenir des globines d'origine animale pour des applications alimentaires.

La demande de brevet japonais 55.008261 déposée le 29 janvier 1980 par Y. SATO et S. HAYAKAWA propose une adsorption sélective du pigment de l'hémoglobine à pH acide avec de la CM cellulose. Ce procédé évite l'utilisation d'alcool et de solvants mais présente l'inconvénient de ne pas décolorer suffisamment l'hémoglobine pour des applications thérapeutiques, en raison de la toxicité de l'hème.

Le brevet soviétique N° 25 44365/28-13 du 8 août 1977 déposé par K. A. LOBUNETS décrit une préparation de globine à usage thérapeutique à partir d'un hémolysat de globules rouges humains. Il s'agit d'un traitement utilisant l'eau oxygénée à chaud, ce qui présente l'inconvénient de gravement dénaturer la protéine.

Dans 3 demandes de brevets déposées en Suède

N° 67 407882-5 du 14 juin 1974,
N° 75 13987-3 du 11 Décembre 1975 et
N° 80 02591-9 du 3 avril 1980

P. G. LINDROOS propose différents traitements en solution alcoolique à pH acide à froid (voir brevet français n° 2 351 604). En fonction de la nature et de la concentration des sels ajoutés, la globine précipite ou reste en solution de manière sélective et est séparée des pigments qui s'agglomérent. L'addition d'imidazole ou de molécules apparentées facilite cette séparation. Cependant dans tous les cas, la globine obtenue est trop riche en pigments pour être utilisable en thérapeutique.

Les procédés voisins proposés par P. ESPENAN dans le brevet français N° 81 09890 du 14 mai 1981 ou par J. H. LUIJERINK dans le brevet britannique N° 1 977 278 du 22 décembre 1978 présentent les mêmes inconvénients.

Or, il serait très intéressant de pouvoir disposer de quantités importantes de globine parfaitement dépigmentée et non dénaturée, en particulier pour l'utilisation thérapeutique en tant que substitut du plasma, comme cela à déjà été dit dans:

SAMPLE et coll. *The J. of Lab. and Clin. Medicine (1952), 40,2 206—210*
STRUMIA et coll. *The J. of Lab and Clin. Medicine (1952), 40,2 211—222.*

La présente invention se propose donc de rémédier à ces inconvénients et de fournir un nouveau procédé de préparation de globine à partir d'hémoglobine qui permette dans des conditions industrielles, d'obtenir, de façon économique, une globine extrêmement purifiée et peu ou pas dénaturée.

C'est en particulier un objet de l'invention que de fournir un tel procédé permettant la fabrication industrielle de globines utilisables en thérapeutique.

Un autre objectif est de fournir un procédé qui soit utilisable pour l'obtention de globine à partir d'hémoglobine provenant de sources très différentes pouvant être notamment:

—sang hémolysé placentaire humain;
—autre sang hémolysé humain;
—banques du sang;
—sang animal en provenance d'abattoirs.

Un autre objectif est de permettre de réaliser des globines alimentaires à partir d'hémoglobines animales.

L'hémoglobine à traiter peut se présenter sous forme native (par exemple culots globulaires hémolysés), sous forme purifiée non dénaturée (par exemple par chromatographie par échange d'anions d'un sang placentaire clarifié), ou encore sous forme de précipité dénaturé (par exemple par précipitation sélective au chloroforme ou au caprylate de sodium—voir LIAUTAUD et coll. Developments in Biological Standardization Vol. 27 1974 pages 107—114; ou bien STEINBUCH et coll. Revue Franc. études chim. et Biol. 1969, XIV, 10, pages 1054—1058).

L'invention a pour objet un procédé de préparation de globine à partir d'hémoglobine dans lequel on prépare une solution alcoolique d'hémoglobine à pH acide, caractérisé en ce que l'on adsorbe sur le charbon actif une solution alcoolique d'hémoglobine à pH acide, de préférence à température ambiante.

De façon surprenante le charbon actif, alors qu'il est connu pour adsorber fortement les protéines, fixe l'hème et n'adsorbe pas la globine. Le rendement en globine est proche de 100 %.

De préférence, la concentration d'alcool, tel que par exemple de l'éthanol ou du méthanol, est d'au moins 40 % et de préférence de 75 à 80 %.

Dans le cas où l'hémoglobine est disponible sous forme d'un précipité, il est avantageux de la reprendre en solution à environ 10 % (poids/volume). La reprise peut par exemple s'effectuer par addition d'eau (20 % du volume total), acidification par HCl 3N puis addition des 80 % d'éthanol.

De préférence, le pH acide est inférieur à 4 en étant de préférence supérieur à 2, par exemple voisin de 3. Lorsque le pH augmente, notamment au delà de 4, le charbon actif commence à adsorber des quantités non négligeables de protéines. De même pour des pH trop acides, le taux de protéines récupérées baisse, une dénaturation de la globine étant également à craindre.

Le choix du charbon actif s'effectue en mesurant le pouvoir de décoloration de l'hémoglobine. Ce pouvoir est estimé dans le rapport R=D.O. 280 nm/D.O. 403 nm. Ce rapport est de 0,26 pour l'hémoglobine non dépigmentée. Les charbons actifs en poudre sont particulièrement préférés et le tableau apparaissant dans la description fournira les exemples des charbons commerciaux actuellement disponibles qui donnent de très bons résultats.

De préférence, on utilise une quantité de charbon actif optimale aussi faible que possible pour minimiser les manipulations. Le rapport du poids de charbon par rapport au poids d'hémoglobine varie selon que l'hémoglobine est à l'état natif en solution ou dénaturé sous forme de précipité. Il augmente dans ce dernier cas et peut être par exemple de l'ordre de 3 g de charbon pour 10 g de précipité d'hémoglobine.

Le temps de contact est de préférence de l'ordre de 15 heures. Ce temps peut grandement varier et est déterminé notamment par des considérations industrielles de production ainsi que de taille et de nombre de cuves.

La force ionique est ajustée à une valeur assez faible, par exemple inférieure à NaCl 0,03 M.

La température n'est de préférence pas supérieure à 20°C afin d'éviter les vapeurs d'alcool pour des raisons de sécurité.

De façon particulièrement avantageuse, on peut procéder à une agitation pendant toute la période de contact, l'agitation augmentant sensiblement le rendement.

L'invention a également trait aux globines industrielles purifiées obtenues. L'indice de décoloration D.O. 280 nm/D.O. 403 nm peut être supérieur à 10 et la globine humaine est apte aux applications thérapeutiques.

D'autres avantages et caractéristiques de l'invention apparaîtront à la lecture de la description suivante faite à titre d'exemple non limitatif.

Exemple 1

Un précipité d'hémoglobine dénaturée est obtenu à partir de sang hémolysé placentaire par précipitation au chloroforme. Le précipité est repris par addition d'eau (20 % du volume total), puis acidification par HCl 3N, après quoi on ajoute l'éthanol nécessaire pour obtenir une concentration à 80 %. Le volume le plus faible pour obtenir la redissolution correspond à une reprise à 10 % (poids/volume); c'est ce volume que est préféré puisqu'il entraîne la plus faible consommation d'alcool. La fraction insoluble est d'environ 33 % du précipité initial (dans le cas d'un précipité au caprylate de sodium, le taux de reprise est de 6 % et la fraction insoluble restante de 25 % du précipité initial).

Le pH de la solution d'hémoglobine est ajusté à 3,3.

Le charbon actif choisi est le charbon type L 4S préparé par la société de nationalité française CECA.

On ajoute à la solution d'hémoglobine à 20°C le charbon actif à raison de 3 g pour 10 g de précipité d'hémoglobine initial (3,4 g pour 10 g dans le cas du précipité au caprylate).

Le mélange effectué dans une cuve, est agité en continu pendant 15 heures, le pH apparent étant de 3,3.

On évacue ensuite le charbon par centrifugation. La solution alcoolique de globine qui forme le surnageant est ensuite diluée dans un volume égal d'eau et neutralisée par addition de soude N. Après précipitation et récupération du précipité de globine par centrifugation ou filtration, on note que le liquide obtenu est parfaitement limpide et incolore et se prête parfaitement aux opérations de recyclage de l'alcool par distillation.

Le précipité de globine peut être ensuite transformé en solution de globine parfaitement décolorée par des procédés déjà connus.

**0 134 729**

Exemple 2

A partir d'une source purifiée d'hémoglobine, par exemple de l'hémoglobine de sang hémolysé placentaire dont on a extrait les immunoglobulines, l'albumine et éventuellement d'autres composants plasmatiques, ou encore à partir d'une solution d'hémoglobine obtenue par lyse d'un culot, on effectue une reprise de l'hémoglobine dans un mélange éthanol-acide de façon qu'en fin d'addition le taux d'éthanol soit de 80 % et le pH apparent de 3,1. Aucun précipité n'apparaît et la solution d'hémoglobine est parfaitement limpide.

On ajoute à la solution 0,2 % en poids/volume de charbon actif L4S. On agite le mélange pendant 15 heures à une température de 20°C. On élimine ensuite le charbon actif par filtration, par exemple à l'aide de terre filtrante ou encore par centrifugation.

La solution alcoolique de globine acide qui apparaît est complètement décolorée. Elle est ensuite diluée avec un volume égal d'eau et neutralisée par addition de soude normale. Après précipitation et récupération du précipité de globine par centrifugation ou filtration, on constate que le liquide obtenu est parfaitement limpide et incolore et se prête parfaitement aux opérations de recyclage de l'alcool par distillation.

La globine obtenue est totalement dépigmentée et peut être transformée en dérivés thérapeutiques par des procédés déjà décrits.

Comparaison des charbons actifs

Les charbons actifs en poudre suivants ont été essayés.

| Dénomination<br>Propriétés | Darco<br>KB | Degussa<br>E114 | Ceca<br>WNCL | Ceca<br>L3S | Ceca<br>L4S |
|---|---|---|---|---|---|
| Teneur en eau % | 25 | <10 | 5 | <5 | <5 |
| Teneur en cendres %<br>$CaCO_3$, $K_2CO_3$, Si | 1,3 | <6,5 | <2 | <3,5 | <2 |
| Valeur du pH dans 20<br>parties d'$H_2O$ distillée | 5 | 9—10 | 4,5 | >4,5 | >4,5 |
| Surface selon BET $m^2/g$ | 1450 | 750 | 1200 | 1200 | 1400 |
| Indice de mélasse | 190 | 100 | — | 80 | 90 |
| Indice bleu méthylène | — | >10 | 20 | 18 | >20 |
| Taille des particules<br>de charbon | 99%<100<br>mesh<br>70%<325<br>mesh | 80%<40µ | 95%<80µ | 90%<80µ | 90%<80µ |
| Densité | 0,110 | 0,365 | 0,580 | 0,200 | 0,180 |

L'efficacité comparée de ces charbons actifs apparaît dans le tableau suivant, en étant déterminée par l'indice R.

| Dénomination | Darco<br>KG | Degussa<br>E114 | Ceca<br>WNCL | Ceca<br>L3S | Ceca<br>L4S |
|---|---|---|---|---|---|
| $R=\dfrac{D.O.\ 280\ nm}{D.O.\ 403\ nm}$ | 13 | 8,2 | 6,5 | 12,7 | >15 |

Le charbon L 4S apparaît comme le plus indiqué. La quantité de charbon est avantageusement de l'ordre de 3 g pour 10 g de précipité d'hémoglobine et de préférence inférieure à 6 g.

A titre d'exemple, une quantité de 10 tonnes de placentas peut fournir:

— soit environ 2,5 tonnes de précipité d'hémoglobine dénaturée par le chloroforme ou le caprylate de sodium,

— soit environ 250 kg d'hémoglobine en solution aqueuse.

4

**0 134 729**

Ces données chiffrées du procédé préférentiel sont résumées dans le tableau final:

| Quantité initiales de matière première | 2,5 tonnes de précipité d'hémoglobine dénaturée | 250 kg d'hémoglobine native |
|---|---|---|
| Volume d'éthanol nécessaire | 30.000 l | 30.000 l |
| Quantité de charbon L 4S | 800 kg | 60 kg |
| Décoloration de la globine $\dfrac{\text{D.O. 280 nm}}{\text{D.O. 403 nm}}$ | 10 | 40 |
| Poids de globine obtenu | 100 kg | 230 kg |

**Revendications**

1. Procédé de préparation de globine à partir d'hémoglobine dans lequel on prépare une solution alcoolique d'hémoglobine à pH acide, caractérisé en ce que l'on adsorbe cette solution sur le charbon actif.

2. Procédé selon la revendication 1, caractérisé en ce que la concentration en alcool est d'au moins 40 %.

3. Procédé selon la revendication 2, caractérisé en ce que la concentration en alcool est de 75 à 80 %.

4. Procédé selon l'une quelconque des revendications 1 à 3, caractérisé en ce que l'on utilise de l'éthanol.

5. Procédé selon l'une quelconque des revendications 1 à 4, caractérisé en ce que le pH est compris entre 2 et 4.

6. Procédé selon la revendication 5, caractérisé en ce que le pH est de l'ordre de 3.

7. Procédé selon l'une quelconque des revendications 1 à 6, caractérisé en ce que le poids de charbon actif est inférieur à 6 g/10 g de précipité d'hémoglobine.

8. Procédé selon la revendication 7, caractérisé en ce que le poids de charbon est de l'ordre de 3 g/10 g de précipité d'hémoglobine.

9. Procédé selon l'une quelconque des revendications 1 à 6, caractérisé en ce que le poids de charbon est de l'ordre de 0,2 % (poids/volume) pour une solution d'hémoglobine native.

10. Procédé selon l'une quelconque des revendications 1 à 9, caractérisé en ce que le charbon actif est un charbon en poudre.

11. Procédé selon la revendication 10, caractérisé en ce que l'on utilise un charbon actif ayant les propriétés suivantes: teneur en eau %<5; teneur en cendre % $CaCO^3$, $K^2CO^3$, Si<2; valeur du pH dans 20 parties d'eau distillée >4,5; surface selon B E T $m^2/g$ 1400; indice de mélasse 90; indice bleu de méthylène <20; taille des particules de charbon 90%<80 microns; densité 0,180.

12. Procédé selon l'une quelconque des revendications 1 à 11, caractérisé en ce que l'on agite le mélange pendant l'adsorption.

13. Procédé selon l'une quelconque des revendications 1 à 12, caractérisé en ce que l'on sépare ensuite le charbon de la globine par centrifugation ou filtration.

14. Procédé selon l'une quelconque des revendications 1 à 13, caractérisé en ce que l'adsorption dure une quinzaine d'heures.

15. Procédé selon l'une quelconque des revendications 1 à 14, caractérisé en ce qu'il est mis en oeuvre à une température de l'ordre de 20°C.

16. Procédé selon l'une quelconque des revendications 1 à 15, caractérisé en ce que la force ionique est ajustée à une valeur assez faible, notamment inférieure à NaCl 0,03 M.

17. Globine obtenue par le procédé selon l'une quelconque des revendications 1 à 16, caractérisée en ce qu'elle est dépigmentée, avec un rapport R=D.O. 280 nm/D.O. 403 nm supérieur à 10.

**Patentansprüche**

1. Verfahren zur Darstellung von Globin aus Hämoglobin, wobei eine alkoholische Hämoglobin-Lösung mit saurem pH-Wert hergestellt wird, dadurch gekennzeichnet, daß diese Lösung an Aktivkohle adsorbiert wird.

2. Verfahren nach Anspruch 1, dadurch gekennzeichnet, daß die Alkohol-Konzentration wenigstens 40% beträgt.

5

3. Verfahren nach Anspruch 2, dadurch gekennzeichnet, daß die Alkohol-Konzentration im Bereich von 75 bis 80 % liegt.

4. Verfahren nach einem der Ansprüche 1 bis 3, dadurch gekennzeichnet, daß Äthanol verwendet wird.

5. Verfahren nach einem der Ansprüche 1 bis 4, dadurch gekennzeichnet, daß der pH-Wert zwischen 2 und 4 liegt.

6. Verfahren nach Anspruch 5, dadurch gekennzeichnet, daß der pH-Wert in der Größerordnung von 3 liegt.

7. Verfahren nach einem der Ansprüche 1 bis 6, dadurch gekennzeichnet, daß das Gewicht der Aktivkohle niedriger als 6 g/10 g Hämoglobin-Präzipitat ist.

8. Verfahren nach Anspruch 7, dadurch gekennzeichnet, daß das Gewicht der Aktivkohle in der Größenordnung von 3 g/10 g Hämoglobin-Präzipitat liegt.

9. Verfahren nach einem der Ansprüche 1 bis 6, dadurch gekennzeichnet, daß das Gewicht der Aktivkohle in der Größenordnung von 0,2% (Gewicht/Volumen) für eine native Hämoglobin-Lösung ist.

10. Verfahren nach einem der Ansprüche 1 bis 9, dadurch gekennzeichnet, daß die Aktivkohle ein Kohlepulver ist.

11. Verfahren nach Anspruch 10, dadurch gekennzeichnet, daß Aktivkohle mit folgenden Eigenschaften verwendet wird: Wassergehalt in % <5; Aschegehalt in % $CaCO^3$, $K^2CO^3$, Si<2; pH-Wert in 20 Teilen destilliertes Wasser >4,5; Oberfläche nach B E T 1400 $m^2$/g; Melasse-Index 90; Methylenblau-Index >20; Größe der Kohleteilchen 90% <80 Mikron; Dichte 0,180.

12. Verfahren nach einem der Ansprüche 1 bis 11, dadurch gekennzeichnet, daß die Mischung während der Adsorption einer bewegenden Einwirkung unterworfen wird.

13. Verfahren nach einem der Ansprüche 1 bis 12, dadurch gekennzeichnet, daß hernach die Kohle vom Globin durch Zentrifugieren oder Filtration getrennt wird.

14. Verfahren nach einem der Ansprüche 1 bis 13, dadurch gekennzeichnet, daß die Adsorption über eine Dauer von etwa fünfzehn Stunden durchgeführt wird.

15. Verfahren nach einem der Ansprüche 1 bis 14, dadurch gekennzeichnet, daß es bei einer Temperatur in der Größenordnung von etwa 20 Grad C durchgeführt wird.

16. Verfahren nach einem der Ansprüche 1 bis 15, dadurch gekennzeichnet, daß die ionische Stärke auf einen genügend schwachen Wert eingestellt wird, insbesondere unterhalb von 0,03 M NaCl.

17. Globin, erhalten durch ein Verfahren nach einem der Ansprüche 1 bis 16, dadurch gekennzeichnet, es depigmentiert ist, mit einem Bezugswert R=D.O. 280 nm/D.O. 403 nm höher als 10.

## Claims

1. Process for the preparation of globin from haemoglobin in which there is prepared an alcoholic solution of haemoglobin at an acid pH, characterised in that this solution is adsorbed on active charcoal.

2. Process according to Claim 1 characterised in that the alcohol concentration is at least 40%.

3. Process according to Claim 2 characterised in that the alcohol concentration is from 75 to 80%.

4. Process according to any one of Claims 1 to 3 characterised in that ethanol is used.

5. Process according to any one of Claims 1 to 4 characterised in that the pH is between 2 and 4.

6. Process according to Claim 5 characterised in that the pH is about 3.

7. Process according to any one of Claims 1 to 6 characterised in that the weight of active charcoal is less than 6 g/10 g of precipitated haemoglobin.

8. Process according to Claim 7 characterised in that the weight of charcoal is about 3 g/10 g of precipitated haemoglobin.

9. Process according to any one of Claims 1 to 6 characterised in that the weight of charcoal is about 0.2% (weight/volume) for a solution of native haemoglobin.

10. Process according to any one of Claims 1 to 9 characterised in that the active charcoal is powdered charcoal.

11. Process according to Claim 10 characterised in that an active charcoal having the following properties is used: water content %<5; ash content %$CaCO_3$, $K_2CO_3$, Si<2; pH value in 20 parts of distilled water >4.5; surface area (according to BET) $m^2$/g 1400; molasses index 90; methylene blue index >20; size distribution of charcoal particles 90%<80 microns; density 0.180.

12. Process according to any of Claims 1 to 11 characterised in that the mixture is agitated during the adsorption.

13. Process according to any of Claims 1 to 12 characterised in that the charcoal and the globin are subsequently separated by centrifugation or filtration.

14. Process according to any of Claims 1 to 13 characterised in that the adsorption lasts for about fifteen hours.

15. Process according to any of Claims 1 to 14 characterised in that it is carried out at a temperature of about 20°C.

16. Process according to any of Claims 1 to 15 characterised in that the ionic strength is adjusted to a sufficiently low value, particularly less than 0.03 M NaCl.

17. Globin obtained by a procedure according to any of Claims 1 to 16, characterised in that it is depigmented, with an R value=O.D.280 nm/O.D. 403 nm of greater than 10.